(19) Europäisches Patentamt
European Patent Office
Office européen des brevets

(11) **EP 1 630 558 B1**

(12) **EUROPÄISCHE PATENTSCHRIFT**

(45) Veröffentlichungstag und Bekanntmachung des Hinweises auf die Patenterteilung:
**12.09.2007 Patentblatt 2007/37**

(51) Int Cl.:
*G01N 33/569* (2006.01)  *G01N 33/58* (2006.01)
*G01N 33/543* (2006.01)

(21) Anmeldenummer: **05017002.6**

(22) Anmeldetag: **04.08.2005**

(54) **Diagnostisches Testsystem für den Nachweis von Antikörpern gegen Erreger von akuten respiratorischen Infektionen und atypischen Pneumonien**

Diagnostic multiplexed bead assay for identification of antibodies against acute respiratory infections and atypical pneumonia

Essai diagnostic à base de microsphères destiné à la détection d' anticorps dirigés contre les infections respiratoires aigües et la pneumonie atypique

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **27.08.2004 DE 102004041659**

(43) Veröffentlichungstag der Anmeldung:
**01.03.2006 Patentblatt 2006/09**

(73) Patentinhaber: **Virion / Serion GmbH**
**97076 Würzburg (DE)**

(72) Erfinder:
• **Hermann, Gerhard, Dr.**
**97076 Würzburg (DE)**
• **Roeschard, Jacqueline, Dr.**
**97218 Gerbrunn (DE)**

(74) Vertreter: **Rudolph, Ulrike**
**Patentanwaltskanzlei Dr. U. Rudolph**
**In der Schanz 10**
**D-69198 Schriesheim (DE)**

(56) Entgegenhaltungen:
**EP-A- 0 126 450**

• MARTINS THOMAS B: "Development of internal controls for the Luminex instrument as part of a multiplex seven-analyte viral respiratory antibody profile" CLINICAL AND DIAGNOSTIC LABORATORY IMMUNOLOGY, Bd. 9, Nr. 1, Januar 2002 (2002-01), Seiten 41-45, XP002350908 ISSN: 1071-412X

• GENDREL D ET AL: "COMPARISON OF PROCALCITONIN WITH C-REACTIVE PROTEIN, INTERLEUKIN 6 AND INTERFERON-ALPHA FOR DIFFERENTIATON OF BACTERIAL VS. VIRAL INFECTIONS" PEDIATRIC INFECTIOUS DISEASE JOURNAL, WILLIAMS & WILKINS, BALTIMORE, MD, US, Bd. 18, Nr. 10, 1999, Seiten 875-881, XP000974949 ISSN: 0891-3668

• VAN DISSEL J T: "Procalcitonin and other markers of infection. What should be their role in clinical practice?" CLINICAL MICROBIOLOGY AND INFECTION : THE OFFICIAL PUBLICATION OF THE EUROPEAN SOCIETY OF CLINICAL MICROBIOLOGY AND INFECTIOUS DISEASES. FEB 2002, Bd. 8, Nr. 2, Februar 2002 (2002-02), Seiten 70-73, XP002350942 ISSN: 1198-743X

• CHAUHAN S ET AL: "Alterations in immunoglobulin & complement levels in chronic obstructive pulmonary disease." INDIAN JOURNAL OF MEDICAL RESEARCH, INDIAN COUNCIL OF MEDICAL RESEARCH, NEW DEHLI, IN, Bd. 92, August 1990 (1990-08), Seiten 241-245, XP008054626 ISSN: 0019-5340

• BALDACCI S ET AL: "Allergy markers in respiratory epidemiology" EUROPEAN RESPIRATORY JOURNAL, Bd. 17, Nr. 4, April 2001 (2001-04), Seiten 773-790, XP002350943 ISSN: 0903-1936

• OLDACH D W ET AL: "RAPID DIAGNOSIS OF CHLAMYDIA PSITTACI PNEUMONIA" CLINICAL INFECTIOUS DISEASES, THE UNIVERSITY OF CHICAGO PRESS, CHICAGO, IL, US, Bd. 17, Nr. 3, 1993, Seiten 338-343, XP008054652 ISSN: 1058-4838

- MC. PROBST ET AL.: "Bead-based multiplex analysis" LABORATORIUMSMEDIZIN 2003 GERMANY, Bd. 27, Nr. 5-6, Juni 2003 (2003-06), Seiten 182-187, XP002342506
- PICKERING J W ET AL: "A MULIIPLEXED FLUORESCENT MICROSPHERE IMMUNOASSAY FOR ANTIBODIES TO PNEUMOCOCCAL CAPSULAR POLYSACCHARIDES" AMERICAN JOURNAL OF CLINICAL PATHOLOGY, PHILADELPHIA, PA, US, Bd. 117, Nr. 4, 2002, Seiten 589-596, XP008054569 ISSN: 0002-9173
- DATABASE MEDLINE [Online] US NATIONAL LIBRARY OF MEDICINE (NLM), BETHESDA, MD, US 20 Mai 1998 LINDBAEK M.; HJORTDAHL P.: ' [C-reactive protein in general practice. An important diagnostic tool in infections]'
- DATABASE BIOSIS [Online] BIOSCIENCES INFORMATION SERVICE, PHILADELPHIA, PA, US 1998 RITTER E.; BUERGER W.; SCHMIDT G.: 'SERUM CONCENTRATION OF C-REACTIVE PROTEIN IN DAY-NURSERY CHILDREN SUFFERING FROM ACUTE RESPIRATORY DISEASES'
- DATABASE BIOSIS [Online] BIOSCIENCES INFORMATION SERVICE, PHILADELPHIA, PA, US Dezember 1998 KUSUNOKI TAKASHI; WRIGHT SAMUEL D.; INOUE YASUHIRO; MIYANOMAE TAKESHI; YOSHIDA YOKO; YONEDA KOZO: 'Serum levels of soluble CD14 in allergic inflammation'

## Beschreibung

Die Erfindung betrifft ein diagnostisches Testsystem für den Nachweis von Antikörpern gegen Erreger von akuten respiratorischen Infektionen und atypischen Pneumonien, das wenigstens einen Erreger von akuten respiratorischen Infektionen und atypischen Pneumonien oder Teile (Proteine, Proteinfragmente, Nukleinsäuren, Nukleinsäurefragmente) dieses Erregers als Sonden- bzw. Detektor-Antigen aufweist.

Atypische Pneumonien werden durch verschiedene Mikroorganismen, insbesondere Viren und Bakterien hervorgerufen und unterscheiden sich in den Beschwerden und im Verlauf von der 'klassischen' Lungenentzündung. Die klassische Lungenentzündung (Pneumonie) ist eine akute oder chronische Entzündung des Lungengewebes, die von Pneumokokken (andere Bezeichnung für Streptokokkus pneumoniae) verursacht wird. Zeigt eine Pneumonie nicht die Zeichen einer klassischen (typischen) Pneumonie, wie sie durch Pneumokokken (Streptokokkus pneumoniae) verursacht wird, dann wird sie als atypisch (untypisch) bezeichnet.

Die atypische Pneumonie wird zunehmend häufiger beobachtet. In Deutschland sind bereits über 20 Prozent der Pneumonien, die im normalen Umfeld des Patienten erworben wurden (sogenannte ambulant erworbene Pneumonien), durch atypische Erreger verursacht. Atypische Pneumonien entstehen meist durch ebenfalls als atypisch bezeichnete Erreger, nämlich durch Legionellen (insbesondere Legionella spp.), Mykoplasmen (insbesondere Mykoplasma pneumoniae), Chlamydien (insbesondere Chlamydia pneumoniae und Chlamydia psittaci) Coxiellen (insbesondere Coxiella burnettii) und Rickettsien, sowie Viren, wie z.B. RSV, Parainfluenza, Influenza oder Adenovirus.

Obwohl es sich bei der atypischen Pneumonie um eine Erkrankung der Lunge handelt, können sich viele der charakteristischen Erkrankungszeichen außerhalb der Lunge zeigen, wie zum Beispiel Hautausschläge oder Gelenkschmerzen. Auch die Legionellenpneumonie (Legionärskrankheit) gehört zu den atypischen Pneumonien. Häufig kann eine eindeutige Diagnose deshalb nur durch einen Nachweis des Erregers im Blut, Sputum, oder einer anderen Körperflüssigkeit oder einem anderen Gewebe des Patienten (bei Legionellen beispielsweise auch im Urin des Patienten) festgestellt werden. Der Verlauf einer atypischen Pneumonie kann sehr unterschiedlich sein. Neben leichten Erkrankungen mit schneller und vollständiger Heilung kommen auch heutzutage noch tödliche Verläufe vor.

Ein Problem bei den atypischen Pneumonien stellt immer noch der teilweise schwierige Erregernachweis zu Beginn der Erkrankung dar.

- Unter den Chlamydien sind vor allem Chlamydia psittaci und Chlamydia pneumoniae als Verursacher einer atypischen Pneumonie bekannt. Beide rufen ähnliche Krankheitssymptome hervor. C.hlamydia psittaci-Infektionen verlaufen jedoch oft schwerwiegender und können vor allem bei verzögertem Behandlungsbeginn oder bei Nichtbehandlung zu lebensbedrohenden Pneumonien führen. Eine Infektion mit C. psittaci wird serologisch nicht erkannt, wenn lediglich auf C. pneumoniae getestet wird. Die Unterscheidung der beiden Erreger ist deshalb sowohl für die Behandlung als auch für die Vermeidung von Rückfällen von großer Bedeutung.
- Ein weiteres Problem beim Nachweis von C. pneumoniae und/oder C. psittaci als mutmaßliche Erreger einer atypischen Pneumonie besteht darin, daß Antikörper gegen Chlamydia trachomatis (einem der in Europa häufigsten Erreger von Infektionen des Genitaltraktes) mit C. pneumoniae und C. psittaci kreuzreagieren. Bei fast allen herkömmlichen Nachweistests, die ein C. pneumoniae-positives Ergebnis zeigen, bleibt daher die Frage offen, ob ein echt-positives Ergebnis vorliegt oder ob es sich um eine Infektion mit C. trachomatis handelt. Erschwert wird eine exakte Diagnose außerdem dadurch, dass C. trachomatis-Infektionen häufig subklinisch verlaufen und die Antikörpertiter lange Zeit bestehen bleiben können.

Im Stand der Technik ist bisher nur ein serologischer Assay bekannt, in dem diese drei Chlamydiaspezies in Kombination eingesetzt werden (Firma Savyon, Vertrieb in Deutschland durch die Firma Hain). Bei diesem Assay handelt es sich um einen Immunfluoreszenztest, bei dem ein Objektträger für jede der drei Chlamydiaspezies je eine Reihe antigenbeschichteter Vertiefungen aufweist. In jeder Reihe muss wenigstens eine der Vertiefungen (Kavitäten) mit einer Probe desselben Patientenserums beschickt werden, damit über ein fluoreszenzbasiertes Verfahren vorhandene Antikörper nachgewiesen werden können. Da für einen Nachweistest wenigstens drei Kavitäten beschickt werden müssen, besteht auch die Gefahr von Fehlerquellen dreifach, und ein direkter Vergleich der Ergebnisse ist nur mit Einschränkungen möglich.

- Bei C. pneumoniae besteht ferner das Problem, daß dieser Erreger chronische Infektionen verursachen kann. Um zwischen einer Erstinfektion und einer chronisch rezidiven Infektion unterscheiden zu können, ist oft die Bestimmung des IgA-Titers, zusätzlich zum IgM- und IgG-Titer das einzige aussagekräftige Diagnoseverfahren. Ein positiver und/oder erhöhter IgM-Titer für sich betrachtet ist ein sicherer Hinweis auf akute Infektionen, fehlt jedoch häufig bei Reinfektionen (v. a. bei viralen respiratorischen Erregern). Liegt hingegen eine abgelaufene Infektion oder ein spätes Infektionsstadium vor, ist häufig ein positiver IgG-Befund der einzige Hinweis auf den Infektionserreger. Erst der gleichzeitige Nachweis der drei verschiedenen Ig-Klassen (IgM, IgA, IgG) liefert ausreichend Informationen für eine umfassende Einschätzung des Infektionsstatus.

- Darüber hinaus liefern die Ergebnisse einer Gesamt-Titerbestimmung von IgA den Hinweis, ob die respiratorische Infektion durch einen Immundefekt bedingt ist, und Gesamt-IgE kann einen Hinweis geben, ob die respiratorischen Symptome durch Allergien bedingt sind.

- Im Fall von Coxiella-Infektionen muss der Arzt zwischen der akuten und der chronisch-rezidiven Form unterscheiden können, da sich die Therapien unterscheiden. Bei der akuten Erkrankung bildet das Immunsystem des Patienten vor allem Antikörper gegen das Phase-II-Antigen, während bei einer chronischen Erkrankung vor allem hohe Titer von anti-Phase-I-Antikörpern im Patientenserum zu finden sind.

- Ein möglichst eindeutiger und vollständiger Erregernachweis zu Beginn der Erkrankung ist erforderlich, um rasch mit einer möglichst gezielten Therapie beginnen zu können. Wichtigste Maßnahme zur Behandlung von atypischen Pneumonien ist die Gabe von geeigneten Antibiotika. Da aber gerade bei atypischen Pneumonien der Erregernachweis mit den herkömmlichen Verfahren oft nicht einfach ist, und nur ein eindeutiger und vollständiger Erregernachweis eine gezielte spezifische Therapie ermöglicht, werden in der Praxis zwangsläufig die Antibiotika nicht selten auf Verdacht gegeben. Gegen die Erreger der bakteriellen atypischen Pneumonie sind im Wesentlichen drei Gruppen von Antibiotika wirksam: die Tetrazykline, die Makrolidantibiotika und die sogenannten Gyrase-Hemmer. Im Fall von C. psittaci und C. pneumoniae sind jedoch Hinweise vorhanden, dass Makrolide zwar für die Behandlung von C. pneumoniae, aber nicht für die von C. psittaci geeignet sind (Quelle Epidemiologisches Bulletin, RKI, 18/97). Eine sichere Unterscheidung der beiden Erreger ist deshalb erforderlich, um fehlerhafte (und damit unwirksame) Therapien zu vermeiden.

Ebenso wichtig ist die Unterscheidung zwischen viralen und bakteriellen Erregern, denn im Fall einer viralen Infektion sind Antibiotika therapeutisch wirkungslos, belasten im Fall einer Einnahme aber den Patientenkörper mit ihren Nebenwirkungen. Von der atypischen Pneumonie müssen deshalb vor allem die durch Viren bedingten Erkältungskrankheiten und die akute Bronchitis abgegrenzt werden. Bisher kann die echte Viruspneumonie oft nur im Verlauf zuverlässig diagnostiziert werden.

Antikörpertiter können sehr lange persistieren. Beispielsweise können Antikörper gegen Chlamydia ssp bis zu 12 Monaten auf hohem Niveau persistieren. Mit den herkömmlichen Tests ist es in solchen Fällen schwierig zu entscheiden, ob ein hoher Antikörpertiter durch eine frische oder eine länger zurück liegende Infektion verursacht ist. Zur Klärung dieser Frage kann in einem parallelen Testlauf ein Marker für akute Infektionen, z.B. Neopterin oder CRP (=C-reaktives Protein), nachgewiesen und quantitativ analysiert werden. Die Neopterinspiegel steigen in frühen Infektionsstadien, noch vor der Serokonversion, bei Infektionen durch Viren, Protozoen und intrazellulären Bakterien an. Mit der Bestimmung des Neopteringehalts im Patientenserum erhält man folglich eine zusätzliche, von spezifischen Antikörpertitern unabhängige Information darüber, ob eine akute virale und/oder bakterielle Infektion vorliegt oder nicht. Für die Differentialdiagnose, ob eine bakterielle oder eine viral bedingte Infektion vorliegt, eignet sich die Analyse des Procalcitonin-Titers oder des CRP-Titers im Patientenserum. Procalcitonin ist ein selektiver Marker für bakterielle Infektionen und zudem wesentlich sensitiver als die übrigen Routine- Entzündungs- Parameter.

Aus der DE 33 22 373 C2 und der EP 0 126 450 ist ein Testsystem zum simultanen Nachweis mehrerer verschiedener Antigene und/oder Antikörper aus einer Probe beschrieben, bei dem Trägerpartikel anstelle der bekannten ELISA-Kavitäten oder (Immuno-/Wester-/Line-)Blotzonen verwendet werden. Diese Trägerpartikel sind mit Detektor- bzw Sonden-Antikörpern und/oder -Antigenen beschichtet. Die Partikel sind außerdem einzeln oder gruppenweise mit Fluoreszenzfarbstoffen unterschiedlicher Emissionsspektren und/oder mit unterschiedlichen Quantitäten wenigstens eines Fluoreszenzfarbstoffes und/oder durch unterschiedliche Partikelgrößen markiert, so daß letztendlich mehrere Partikelpopulationen (jede einzelne bestehend aus einem oder mehreren gleichartigen Partikeln) vorliegen, von denen jede durch eine für sie spezifische Kodierung, nämlich die Kombination aus Größe, Fluoreszenzfarbstoffart und Fluoreszenzfarbstoffmenge, charakterisiert ist. Jede Partikelpopulation (Partikelset) ist zudem mit einer anderen Art von Detektor- bzw. Sonden-Antikörpern und/oder - Detektor- bzw. Sonden-Antigenen beladen. Eine Mischung solcher Partikelpopulationen wird mit derjenigen Flüssigkeit (z.B. Patientenserum) gemischt, die die zu untersuchenden bzw. nachzuweisenden Antigene bzw. Antikörper (Proben-Antigene bzw. Proben-Antikörper) enthält. Anschließend werden die Reaktionsschritte eines konventionellen Immunfluoreszenzverfahrens durchgeführt: Nach einer definierten Reaktionszeit, in der die nachzuweisenden Antigene bzw. Antikörper von den an die Partikel beschichteten Antikörpern bzw. Antigenen gebunden werden, erfolgt die Identifizierung der gebundenen Antigene bzw. Antikörper durch Zugabe einer Flüssigkeit mit fluoreszenzmarkierten Antikörpern und/oder Antigenen, die mit den nachzuweisenden bzw. zu untersuchenden Antigenen bzw. Antikörpern speziesspezifisch reagieren. Zum Schluß wird mittels eines Meßgerätes, z.B. eines Durchflußzyrtometers, jedes Partikel hinsichtlich seiner Kodierung, nämlich Größe und Fluoreszenz-Emmisionsspektrum und Fluoreszenz-Intensität, vermessen. Anhand dieser Meßdaten kann auf das Vorhandensein der zu untersuchenden Antigene bzw. Antikörper rückgeschlossen werden.

Martins T.B. beschreibt in CLINICAL AND DIAGNOSTIC LABORATORY IMMUNOLOGY, Bd.9, Nr-1, 2002, S.41-45 ein diagnostisches Testsystem für den Nachweis von Antikörpern gegen sieben ausschließlich virale Erreger von akuten respiratorischen Infektionen, nämlich Influenza A und B, Adenovirus, Parainfluenza 1,2,3 und RSV. Die Antigene sind an Trägerpartikel gekoppelt, die gruppenweise verschiedene Fluoreszenzfarbstoffmarkierungen aufweisen, d.h. jede Trägerpartikelpopulation ist durch eine bestimmte Fluorezenzfärbung kodiert ist. Das Testsystem weist zudem eine Fluoreszenzfarbstoff-codierte Trägerpartikelpopulation mit daran gekoppelten Sonden/Markern zum Nachweis von IgG-Antikörpem und eine Fluoreszenzfarbstoff-codierte Trägerpartikelpopulation mit daran gekoppeltem Sonden/Markern zu Nachweis von IgM-Antikorpern auf.

Aus Gendrel et al., Pediatr Infect Dis J, 1999; Vol. 18 No. 10, S.875-881 ist die Eignung von Procalcitonin und C-reaktivem Protein als Marker für die Differenzierung bakterieller und viraler Infektionen bekannt.

Van Dissel, Clin Microbiol Infect 2002; 8: 70-73 beschreibt und vergleicht Infektionsmarker, insbesondere C-reaktives Protein, Procalcitonin und Neopterin.

Chauhan et al., Indian J Med res [B] 92, August 1990, S. 241-245 beschreiben, dass bei Patienten mit COPD (chronic obstructive pulmonary disease) zum Teil erhöhte IgA- (und IgG-) Titer gefunden werden, während die IgM-Titer offenbar unverändert bleiben.

Aus Baldacci et al, Eur Respir J 2001, 17: S. 773-790 ist bekannt, dass Allergien durch Serum IgE bestimmt werden können.

Oldach et al., Clinical Infectious Dieseases 1993; Vol. 17, S. 338-343 beschreiben den Antigennachweis von Chlamydia psitacci mittels eines direkten Immunfluoreszenztests unter Einsatz von IFT und fluoreszenzmarkierten monoklonalen Antikörpern gegen das Antigen.

Der vorliegenden Erfindung liegt die Aufgabe zugrunde, ein Testsystem für die Diagnose von Antikörpern gegen Erreger von akuten respiratorischen Infektionen und atypischen Pneumonien bereitzustellen, das schnell durchzuführen ist, zuverlässige spezifische Ergebnisse liefert, und das den Nachweis und die Identifizierung aller relevanten Antikörper gegen die Erreger (insbesondere Bakterien Viren, Mykoplasmen) von akuten respiratorischen Infektionen und atypischen Pneumonien sowie die Bestimmung des Infektionsstadiums (Erstinfektion, Persistenz, Reaktivierung) in einem und demselben Arbeitsgang ermöglicht.

Eine Lösung dieser Aufgabe besteht in der Bereitstellung eines Testsystems gemäß Ansprüchen 1-14, nämlich eines Testsystems für die Diagnose von Antikörpern gegen Erreger von akuten respiratorischen Infektionen und atypischen Pneumonien, das Erreger von akuten respiratorischen Infektionen und atypischer Pneumonien oder antigene Teile (Proteine, Proteinfragmente, Nukleinsäuren, Nukleinsäurefragmente) dieser Erreger als Sonden- bzw. Detektor-Antigene aufweist, wobei diese Sonden- bzw. Detektorantigene an Trägerpartikel gekoppelt sind, welche einzeln oder gruppenweise markiert sind, so daß mehrere Partikelpopulationen, - jeweils bestehend aus einem oder mehreren gleichartigen Partikel, - vorliegen, von denen jede durch eine für sie spezifische Markierung kodiert ist, und das dadurch gekennzeichnet ist, daß die Erreger ausgewählt sind aus der Gruppe I: Chlamydophila pneumoniae, Chlamydophila psittaci, Mycoplasma pneumoniae, Coxiella burnetii Phase 1 und 2, Legionella pneumophila, Bordetella pertussis, oder aus der Gruppe II. Parainfluenza 1, 2, 3, RSV, Adenovirus, Influenza A und B, Picornaviren, Varizella-Zoster Virus, Masern Virus, daß das Testsystem zudem wenigstens einen Marker für akute Infektionserkrankungen (sogenannter "Akute-Phase-Marker") und wenigstens einen Marker für bakterielle Infektionen aufweist, dass die Marker ebenfalls an Trägerpartikel gekoppelt sind, und dass jede Partikelpopulation mit einer anderen Art Sonden- bzw. Detektorantigen oder Marker beladen ist.

Das erfindungsgemäße Testsystem bietet vor allem den Vorteil, daß auf die Fragen nach dem Vorliegen einer akuten respiratorischen Infektion und/oder einer atypischen Pneumonie und gegebenenfalls nach dem Verlauf und dem Status der Infektion eine aussagekräftige, serologische Antwort anhand eines einzigen Testlaufs mit einer einzigen Serumprobe des betreffenden Patienten erhalten werden kann. Mit den herkömmlichen Testssystemen müßte hierfür eine Mehrzahl von Testläufen (bedingt durch die Vielzahl der in Betracht kommenden spezifischen Erreger, zusätzlich mögliche allergische Reaktionen und/oder die mögliche Überlagerungen von persistierenden und frischen Infektionen) durchgeführt werden. Aufgrund der zunehmenden Kostenbeschränkung im Gesundheitssystem ist jedoch die Anzahl der durchführbaren Tests limitiert, was zu einer - mehr oder weniger willkürlichen - Auswahl einiger weniger Parameter führt. Die mit dem erfindungsgemäßen Testsystem durchführbare gleichzeitige Analyse mehrerer Parameter ermöglicht eine (insbesondere im Vergleich zu den herkömmlichen separaten Untersuchungsgängen) schnellere und kosteneffizientere Diagnose und daraus resultierend eine entsprechend spezifische und wirkungsvolle Therapie.

In einer bevorzugten Ausführungsform des erfindungsgemäßen Testsystems sind als Erreger aus Gruppe I Chlamydophila pneumoniae, Chlamydophila psittaci, Mycoplasma pneumoniae und Bordetella pertussis und zusätzlich Chlamydia trachomatis eingesetzt, vorzugsweise außerdem noch Coxiella burnetii Phase 1 und 2 und Legionella. Dieses Testsystem ist besonders auf den Nachweis von bakteriell bedingten respiratorischen Erkrankungen ausgerichtet. Die Erreger Chlamydophila pneumoniae, Chlamydophila psittaci, Mycoplasma pneumoniae und Bordetella pertussis sowie Viren können primär-atypische Pneumonien auslösen, die aufgrund der Symptomatik allein nicht zu unterscheiden sind. Chlamydophila trachomatis dagegen führt zu Infektionen des Genitaltraktes und zählt zu einer der häufigsten Geschlechtskrankheiten in Europa. Diese Infektionen verlaufen jedoch häufig subklinisch und damit unerkannt und die Antikörpertiter können lange Zeit bestehen bleiben. Antikörper gegen Chlamydophila trachomatis reagieren häufig ebenfalls mit Chlamydophila pneumoniae und Chlamydophila psittaci. Der Nachweis von Antikörpern gegen Chlamydophila trachomatis in dieser erfindungsgemäßen Testsystemvariante gibt einen Hinweis auf eventuelle Kreuzreaktionen und erlaubt eine diagnostische Aussage dahingehend, ob ein Chlamydophila pneumoniae -positives Testergebnis tatsächlich eine Infektion mit Chlamydophila pneumoniae anzeigt, oder ob nicht nur oder auch eine Infektion mit Chlamydophila trachomatis vorliegt.

Infektionen mit Chlamydophila psittaci rufen eine ähnliche Symtomatik hervor wie Infektionen mit Chlamydophila pneumonia, wobei Chlamydophila psittaci-Infektionen oft schwerwiegender verlaufen und vor allem bei verzögertem Behandlungsbeginn oder Nichtbehandlung bis zu lebensbedrohenden Pneumonien führen können. Eine Infektion mit Chlamydophila psittaci wird serologisch nicht erkannt, wenn lediglich auf Chlamydophila pneumonia getestet wird. Die Unterscheidung der beiden Erreger ist jedoch sowohl für die Behandlung als auch für die Vermeidung von Rückfällen von großer Bedeutung. Die Behandlung beider Erreger erfolgt durch Antibiotikagaben. Es sind jedoch Hinweise vorhanden, daß einige Antibiotika für die Behandlung von Chlamydophila pneumonia, nicht dagegen für die Behandlung von Chlamydophila psittaci geeignet sind. Eine sichere Unterscheidung der beiden Erreger ist notwendig, um fehlerhafte und damit unwirksame Therapien zu vermeiden, und mit dem erfindungsgemäßen Testsystem einfach realisierbar.

In einer anderen, ebenso bevorzugten Ausführungsform des erfindungsgemäßen Testsystems sind als Erreger Parainfluenza 1, 2, 3, RSV, Adenovirus, Influenza A und B eingesetzt, weiter vorzugsweise außerdem noch Varizella-Zoster Virus und Masern Virus

Dieses Testsystem ist besonders auf den Nachweis von virusbedingten respiratorischen Erkrankungen ausgerichtet, es ermöglicht eine schnelle Differentialdiagnose und die Vermeidung eines unnötigen Einsatzes von Antibiotika.

In einer noch anderen, gleichfalls bevorzugten Ausführungsform des erfindungsgemäßen Testsystems sind als Erreger aus Gruppe I Chlamydophila pneumoniae, Chlamydophila psittaci, Mycoplasma pneumoniae sowie Bordetella pertussis und als Erreger aus Gruppe II Parainfluenza 1, 2, 3, RSV, Adenovirus sowie Influenza A und B und zusätzlich Chlamydia trachomatis eingesetzt.

Dieses Testsystem ist insbesondere für die Untersuchung von Kleinkindern geeignet, da es die wichtigsten Erreger von Pneumonien bei Kleinkindern umfaßt. Gerade bei Kleinkindern ist eine parallele Austestung mehrere Parameter gewünscht, da nur kleine Mengen Patientenblut abgenommen werden können. Dieses Testsystem ermöglicht eine schnelle Differentialdiagnose, den spezifischen Einsatz von Antibiotika bei bakteriellen Infektionen und die Vermeidung unnötiger Antibiotikatherapien im Fall von viralen Infektionen.

Als Marker für akute Infektionen weist das erfindungsgemäße Testsystem vorzugsweise Neopterin auf. Antikörpertiter gegen respiratorische Infektionserreger können sehr lange persistieren, im Fall von Chlamydophila peneumoniae beispielsweise bis zu 12 Monate auf hohem Niveau. In herkömmlichen Tests ist es daher oft schwierig zu entscheiden, ob ein hoher Antikörpertiter durch eine frische oder eine ältere Infektion bedingt ist. Neopterin ist ein natürliches humanes Stoffwechselabbauprodukt und wird vermehrt gebildet, wenn Infektionen vorliegen. Im Fall von Infektionen durch Viren, Protozoen und/oder intrazellulären Bakterien steigt der Neopterinspiegel schon in frühen Infektionsstadien, noch vor der Serokonversion, deutlich an. Mit der erfindungsgemäßen Bestimmung des Neopterinspiegels erhält man eine differentialdiagnostische Aussage zum Vorliegen von akuten viralen und/oder bakteriellen Infektionen. Als Marker für bakterielle Infektionen wird in dem erfindungsgemäßen Testsystem vorzugsweise Procalcitonin eingesetzt. Procalcitonin ist ein natürliches humanes stoffwechselprodukt und ein selektiver Marker für bakterielle Infektionen. Procalcitonin ist als Indikator für bakterielle Infektionen wesentliche sensitiver, als die übrigen bisher bekannten Routine-Entzündungs-Parameter.

Der erfindungsgemäße Einsatz von Procalcitonin - vorzugsweise- zusammen mit Neopterin - in dem erfindungsgemäßen Testsystem ermöglicht auf einfache Weise eine Unterscheidung zwischen bakteriellen und viral bedingten Infaktionen.

Anstelle von Procalcitonin und/oder Neopterin kann auch CRP (= C-reaktives Protein) eingesetzt werden.

CRP ist zum einen ein Akut-Phase-Marker, der schon innerhalb der ersten Stunden nach einer Infektion ansteigt und nach 24 - 48 Stunden sein Maximum erreicht. Zugleich erlaubt dieser Marker auch die Differentialdiagnose zwischen Infektionen viraler und bakterieller Natur, da CRP bei viralen Infektionen nur schwach, bei bakteriellen hingegen sehr stark ansteigt.

Der Einsatz von Markern für Gesamt-IgA ist dafür vorgesehen, einen gegebenenfalls vorhandenen Immundefekt nachzuweisen. Das ermöglicht eine diagnostische Aussage darüber, ob die respiratorischen Infekte durch einen Immundefekt (mit-) bedingt sind oder nicht.

Der Einsatz von Markern für Gesamt-IgE ist dafür vorgesehen, gegebenenfalls vorhandene Allergien nachzuweisen. Das ermöglicht eine diagnostische Aussage darüber, ob die respiratorischen Symptome durch Allergien (mit-) bedingt sind oder nicht, und ob eine spezifische Therapie angezeigt ist oder nicht. Gegebenenfalls kann eine adäquate Therapie durchgeführt und die unnötige Verabreichung von Antibiotika vermieden werden.

Als Material für die Trägerpartikel hat sich in der Praxis Latex als besonders geeignet erwiesen. Eine bevorzugte Ausführungsform des erfindungsgemäßen Testsystems zeichnet sich folglich dadurch aus, daß die Trägerpartikel in Form von Latexpartikel realisiert sind.

In der praktischen Erprobung hat sich außerdem eine Variante als sehr geeignet erwiesen, bei der mehrere Partikelpopulationen vorliegen, wobei die Partikel aller Populationen die gleiche Partikelgröße, vorzugsweise zwischen 1 $\mu$m und 10 $\mu$m, besitzen und mit demselben Fluoreszenzfarbstoff markiert sind, aber jede Partikelpopulation eine andere Fluoreszenzintensität aufweist.

In einer weiteren, für die praktische Anwendung besonders bevorzugten Ausführungsform umfasst das erfindungsgemäße diagnostische Testsystem zusätzlich (erstens) für jeden nachzuweisenden (d.h. zu den Sondenantigenen komplementären) Antikörper die Angabe einer Referenzkurve, die die Korrelation (das Verhältnis) zwischen Antikörperkonzentration im Probenvolumen und gemessener Fluoreszenzsignalstärke wiedergibt, und (zweitens) ein Standardserum, welches alle nachzuweisenden, d.h. zu den Sondenantigenen komplementären Antikörper in bekannten Konzentrationen enthält. Die Antikörper-spezifischen Referenzkurven sind durch die Formel

$$\text{Count} = A + \frac{D - A}{1 + e^{B(C - \ln \text{Konz.})}}$$

bzw.:

$$\text{Konz.} = \exp\{C - \ln[(D-A)/(\text{Count} - A) - 1]/B\}$$

mit:

Count = Median der gemessenen Fluoreszenzsignale
A = untere Asymptote der Referenzkurve
B = Steigung der Referenzkurve
C = Wendepunkt der Referenzkurve
D = obere Asymptote der Referenzkurve

beschrieben. Diese Referenzkurvenformel ist im Stand der Technik bekannt, beispielsweise aus DE 36 39 279 C3 in Kombination mit Van Loon und Van der Veen in J. Clin. Pathol. 33, 635-639, 1980 und Van Loon et al., J. Clin. Pathol. 34, 1981, 665-669 und aus Dundley, R.A., Edwards, P., Ekins, R.P., Finney, D.J., McKenzie, J.G.M., Raab, G.M., Rodbard, D., and Rodgers, R.P.C. (1985) "Guideline for immunoassay data processing." Clin. Chem. 31, 1264-1271.

Die Referenzkurven-spezifischen Parameter A, B, C, und D werden experimentell mit der dem Fachmann bekannten und geläufigen Methode der Titration (geometrische Verdünnungsreihe) von Kalibratorseren (z.B. internationalen oder nationalen Standards) ermittelt. Vorzugsweise wird für jeden nachzuweisenden Antikörper (Analyten) und für jede seiner Herstellungschargen die Referenzkurve in mehrfachen Testläufen unter Einhaltung optimaler Bedingungen erstellt. Damit geht der Vorteil einher, dass die kosten- und zeitintensive Erstellung solcher Referenzkurven auf Seitens des Anwenders, insbesondere des Arztes oder des Laborpersonals, entfallen kann.

Mit Hilfe des Standardserums werden die Antikörper-spezifischen Referenzkurven rekalibriert, d.h. an die tatsächlichen, aktuellen Testbedingungen angepaßt.

Bei der Rekalibrierung (Tageskorrektur) wird das Verhältnis zwischen Ist-Zustand (IstWert) und Soll-Zustand (Sollwert) ermittelt, das wie folgt in die o.g. Referenzkurvenformel einfließt:

$$\text{Konz.} = \exp\{C - \ln[(D-A)/((\text{Count}-KLW)^* STD_{soll}/(STD_{ist}-KLW) - A) - 1]/B\}$$

mit:

KLW = Konjugatleerwert
$STD_{soll}$ = Sollwert des Standards
$STD_{ist}$ = aktuelle Tageswert des Standards

In der praktischen Anwendung erfolgt die jeweilige Ausrechnung dieser Formel vorzugsweise mit Hilfe von Rechnern und entsprechender Auswertesoftware, so dass die errechneten Ergebnisse der Quantifizierung nahezu zeitgleich mit den tatsächlich ermittelten Messdaten des durchgeführten Diagnosetestlaufs vorliegen.

Mit einer solchen Kalibrierung werden Testschwankungen ausgeglichen und die Qualität des Testlaufes überprüft.

Diese Variante des erfindungsgemäßen Testsystems erlaubt eine quantitative Auswertung der Testergebnisse. Anhand dieser quantitativen Auswertung können Aussagen dazu getroffen werden, ob ein positives Testergebnis tatsächlich eine akute Infektion mit dem betreffenden Erreger anzeigt, oder ob ein Antikörpertiter aufgrund einer früheren Infektion oder einer Kreuzreaktion vorliegt.

Die Erfindung wird im folgenden anhand von Ausführungsbeispielen näher erläutert.

### Beispiel 1: Partikelkodierung

Im Unterschied zur ELISA-Technik, bei der die Kavitätswände der Mikrotiterplatte zur adsorptiven Kopplung der Analyte bzw. Antikörper dienen, werden in dem erfindungsgemäßen Testsystem Trägerpartikel, vorzugsweise Latexpartikel, mit einem Durchmesser von beispielsweise 4,0 μm als Festphase eingesetzt. Die prinzipiell ununterscheidbaren Partikel sind hier im Beispiel mit verschiedenen, distinkten Intensitäten desselben roten Fluorophors gefärbt, wodurch ein Sortiment von sieben unterschiedlich stark rot gefärbten Latexpartikel-Sets (Partikelpopulationen) erhalten wird.

Jedes Set (jede Partikelpopulation) wird mit unterschiedlichen Antigenen beschichtet, das heißt, an jedem Partikelset wird eine bestimmte Art Antigen kovalent oder nicht-kovalent immobilisiert, beispielsweise wie folgt:

Partikelset/Partikelpopulation mit Rot-Intensität 1 : mit Chlamydia trachomatis
Partikelset/Partikelpopulation mit Rot-Intensität 2 : mit Mycoplasma pneumoniae
Partikelset/Partikelpopulation mit Rot-Intensität 3 : mit Bordetella pertussis
Partikelset/Partikelpopulation mit Rot-Intensität 4: mit Parainfluenza 1
Partikelset/Partikelpopulation mit Rot-Intensität 5 : mit Parainfluenza 2
Partikelset/Partikelpopulation mit Rot-Intensität 6: mit Parainfluenza 3
Partikelset/Partikelpopulation mit Rot-Intensität 7: mit RSV
Partikelset/Partikelpopulation mit Rot-Intensität 8: mit Adenovirus,
Partikelset/Partikelpopulation mit Rot-Intensität 9: mit Influenza A
Partikelset/Partikelpopulation mit Rot-Intensität 10: mit Influenza B
Partikelset/Partikelpopulation mit Rot-Intensität 11: mit Neopterin
Partikelset/Partikelpopulation mit Rot-Intensität 12: mit Procalcitonin

Um nun mehrere Untersuchungen gleichzeitig in einem Testansatz durchführen zu können, werden die antigenbeschichteten Partikel-Sets (Partikelpopulationen) zu einem sogenannten Test-Cocktail gemischt. Ein solcher Test-Cocktail (d.h. eine solche konkrete Partikel-Set-Mischung), der (die) beispielsweise die Antigene Chlamydia trachomatis, Mycoplasma pneumoniae, Bordetella pertussis, Parainfluenza 1, 2, 3, RSV, Adenovirus, Influenza A und B (oder geeignetenfalls auch nur Fragmente dieser Antigene) sowie Neopterin und Procalcitonin aufweist, stellt eine Ausführungsvariante des erfindungsgemäßen Testsystems dar. Diese genannte Ausführungsvariante ist vor allem für Untersuchungen an Kleinkindern vorgesehen und geeignet, da es alle wichtigen Erreger von Pneumonien bei Kleinkindern umfaßt.

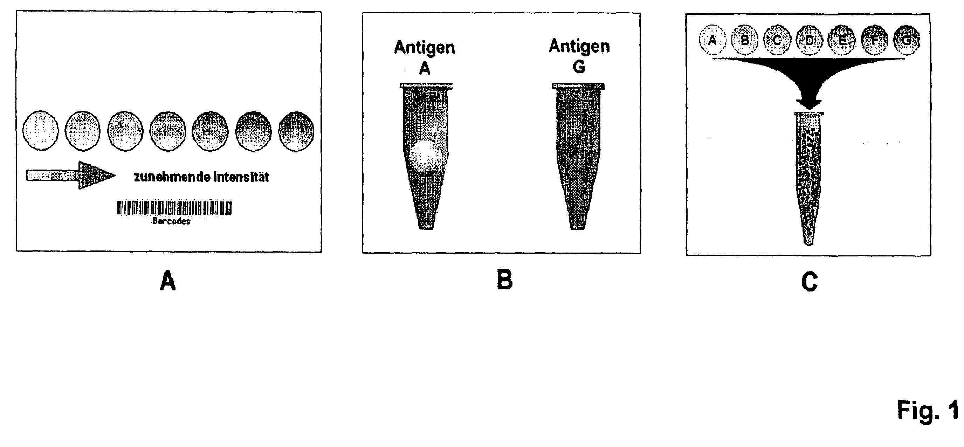

In Fig. 1 sind die einzelnen Schritte der Partikelkodierung und Testsystemherstellung noch einmal bildlich dargestellt. Fig. 1 (A) zeigt die Kodierung der Partikel mit dem roten Fluorophor in verschiedenen Intensitätsstufen. Fig. 1 (B) zeigt die Beschichtung der Partikel mit dem Antigen. Fig. 1 (C) zeigt die Mischung der beschichteten Partikel zu dem Test-Cocktail.

### Beispiel 2: Durchführung eines Tests zur Klärung der Frage, ob ein Patient Antikörper gegen Erreger von akuten respiratorischen Infektionen und atypischen Pneumonien besitzt oder nicht

Eine vorzugsweise gemäß Beispiel 1 hergestellte Partikel-Set-Mischung wird mit einer bestimmten Menge Patientenserum gemischt und inkubiert. Während der Inkubation binden vorhandene Antikörper aus dem Patientenserum ("Patientenantikörper") an diejenigen Latexpartikel, die mit dem ihrer Spezifität entsprechenden Antigen beschichtet sind.

Anschließend wird der Probe (-nlösung) ein Konjugatantikörper zugegeben, beispielsweise ein gegen humane Antikörper gerichteter, polyklonaler Ziegen-Antikörper, der mit dem an die Latexpartikel gebundenen Patientenantikörper reagiert, das heißt an diesen bindet. Dieser Konjugatantikörper ist vorzugsweise mit einem weiteren Fluoreszenzfarbstoff markiert, wobei dieser weitere Fluoreszenzfarbstoff ein anderes Emissionsspektrum oder zumindest eine andere Farbstoffintensität aufweist als der Fluoreszenzfarbstoff, mit dem die Partikel(sets) markiert sind. Hier im Beispiel wurde ein grüner Fluorophor gewählt.

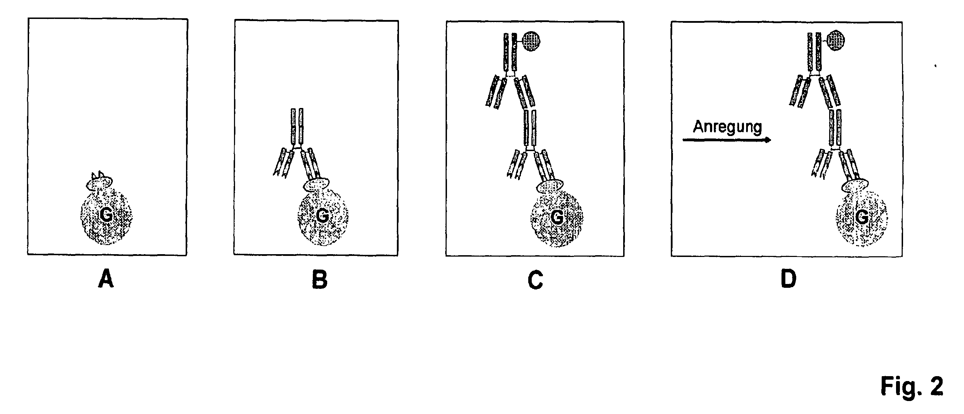

Die Menge gebundener Konjugatmoleküle ist proportional zur Antikörperkonzentration. In Fig. 2 sind die einzelnen Schritte der Testdurchführung noch einmal bildlich dargestellt. Fig. 2 (A) zeigt die Vorlage des Test-Cocktails. Fig. 2 (B) zeigt die Zugabe des Patientenserums mit den Patienten-spezifischen Patientenantikörpern. Fig. 2 (C) zeigt die Zugabe des Konjugatantikörper (mit grüner Fluoreszenz). Fig. 2 (D) zeigt das Reaktionsgefäß während der Detektion im Durchflußzytometer.

### Beispiel 3: Testauswertung

Das vorzugsweise gemäß Beispiel 1 und Beispiel 2 hergestellte und eingesetzte Testsystem trägt nun je Latexpartikel-Set eine variable Menge der Konjugat-Fluoreszenz auf seiner Oberfläche und wird vorzugsweise mit Hilfe eines für Partikel-Messungen spezialisierten Durchflußzytometers, eines sogenannten Partikel-Fluoreszenz-Durchfluß-Meßgeräts, analysiert.

Diese Meßgeräte analysieren individuelle Latexpartikel nach ihrer Fluoreszenz und können gleichzeitig drei verschiedene Fluoreszenzfarben (Orange, Rot, Dunkelrot) unterscheiden.

Hier im Beispiel analysiert das Durchflußzytometer zum einen anhand der Rot-Emission die verschiedenen Partikelsets und damit die verschiedenen Antigene und zum anderen anhand der Grün-Emission die Menge gebundener Konjugat-Antikörper und damit die Menge an gebundenem Patientenantikörper für jedes Partikelset d.h. für jedes spezifische Antigen.

Ein großer Vorteil dieser Durchflußzytometrie-Analytik ist darin zu sehen, daß entsprechende Geräte meist bereits bei den Anwendern vorhanden sind. Darüber hinaus wird die diagnostische Leistungsfähigkeit der immunologischen Teste durch die erhöhte Sensitivität der Fluoreszenz-Signalgebung im Vergleich zur ELISA-Farbreaktion erhöht. Mit Hilfe einer Software klassifiziert das Gerät die Fluoreszenz jedes Partikels zu den passenden Partikel-Sets und ordnet die entsprechenden Analytbestimmungen zu. Die mittlere Konjugat-Fluoreszenz je Partikel-Set ist ein Maß für die Konzentration des entsprechenden Analyten in der Serumprobe.

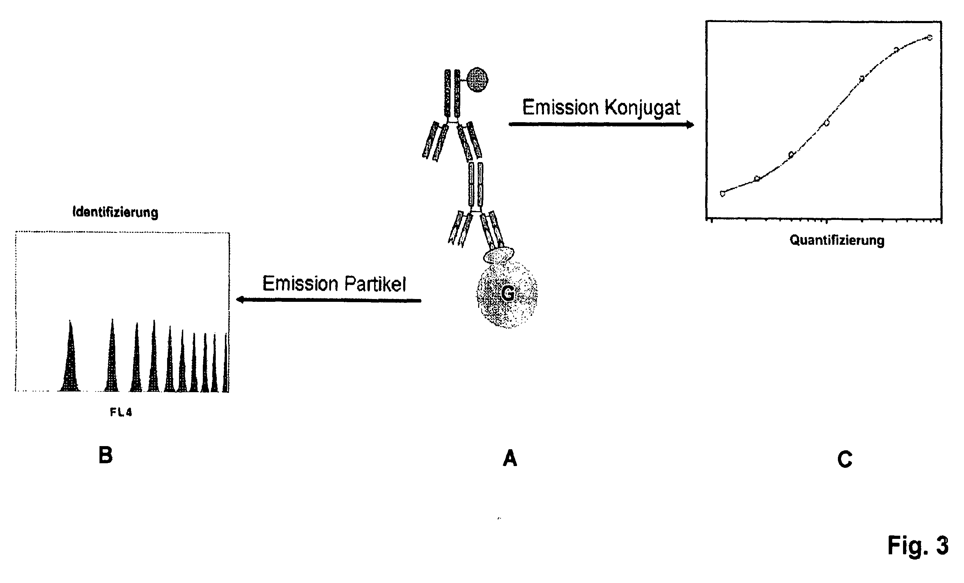

In Fig. 3 sind die einzelnen Schritte der Testauswertung noch einmal bildlich dargestellt. Fig. 3 (A) zeigt das Reaktionsgefäß während der Detektion im Durchflußzytometer. Fig. 3 (B) zeigt das Detektionsbild der roten Emission, und erlaubt damit die Identifikation der verschiedenen, mit spezifischen Antigenen beladenen Partikelpopulationen. Fig. 3 (C) zeigt das Detektionsbild der grünen Emission und erlaubt damit die Identifizierung und Zuordnung der Menge an Konjugatantikörper je Partikelpopulation.

### Patentansprüche

1. Diagnostisches Testsystem für den Nachweis von Antikörpern gegen Erreger von akuten respiratorischen Infektionen und atypischen Pneumonien, das Erreger von akuten respiratorischen Infektionen und atypischen Pneumonien oder antigene Teile dieser Erreger als Sondenantigene aufweist wobei diese Sondenantigene an Trägerpartikel gekoppelt sind, welche einzeln oder gruppenweise markiert sind, so daß mehrere Partikelpopulationen, jeweils bestehend aus einem oder mehreren gleichartigen Partikeln, - vorliegen, von denen jede durch eine für sie spezifische Markierung, nämlich die Kombination aus Größe, Fluoreszenzemissionsspektrum und Fluoreszenzintensität, codiert ist, **dadurch gekennzeichnet,**

   - **daß** die Erreger ausgewählt sind aus der Gruppe I: Chlamydophila pneumoniae, Chlamydophila psittaci, Mycoplasma pneumoniae, Coxiella burnetii Phase 1 und 2, Legionella pneumophila, Bordetella pertussis, oder aus der Gruppe II: Parainfluenza 1, 2, 3, RSV, Adenovirus, Influenza A und B, Picornaviren, Varizella-Zoster Virus, Masern Virus,
   - **daß** das Testsystem zudem wenigstens einen Marker für akute Infektionserkrankungen und wenigstens einen Marker für bakterielle Infektionen aufweist,
   - **daß** die Marker ebenfalls an Trägerpartikel gekoppelt sind,
   - und **dass** jede Partikelpopulation mit einer anderen Art Sondenantigen oder Marker beladen ist.

2. Testsystem nach Anspruch 1, **dadurch gekennzeichnet,**
   **daß** als Erreger aus Gruppe I Chlamydophila pneumoniae, Chlamydophila psittaci, Mycoplasma pneumoniae und Bordetella pertussis und zusätzlich Chlamydia trachomatis eingesetzt sind.

3. Testsystem nach Anspruch 2, **dadurch gekennzeichnet,**
   **daß** es zudem die Erreger Coxiella burnetii Phase 1 und 2 und Legionella pneumophila aufweist.

4.  Testsystem nach Anspruch 1, **dadurch gekennzeichnet,**
    **daß** als Erreger aus Gruppe II Parainfluenza 1, 2, 3, RSV, Adenovirus, Influenza A und B eingesetzt sind.

5.  Testsystem nach Anspruch 4, **dadurch gekennzeichnet,**
    **daß** es zudem die Erreger Varizella-Zoster Virus und Masern Virus aufweist.

6.  Testsystem nach Anspruch 1, **dadurch gekennzeichnet,**
    **daß** als Erreger aus Gruppe I Chlamydophila pneumoniae und/oder Chlamydophila psittaci Mycoplasma pneumoniae sowie Bordetella pertussis und als Erreger aus Gruppe II Parainfluenza 1, 2, 3, RSVsowie Adenovirus Influenza A und B und zusätzlich Chlamydia trachomatis eingesetzt sind.

7.  Testsystem nach einem der Ansprüche 1 bis 6, **dadurch gekennzeichnet, daß** als Marker für akute Infektionen Neopterin oder CRP (=C-reaktives Protein) eingesetzt ist.

8.  Testsystem nach einem der Ansprüche 1 bis 7, **dadurch gekennzeichnet, daß** als Marker für bakterielle Infektionen Procalcitonin oder CRP (=C-reaktives Protein) eingesetzt ist.

9.  Testsystem nach einem der Ansprüche 1 bis 8, **dadurch gekennzeichnet, daß** es zusätzlich wenigstens einen Marker für Gesamt-IgA und/oder wenigstens einen Marker für Gesamt-IgE aufweist.

10. Testsystem nach einem der Ansprüche 1 bis 9, **dadurch gekennzeichnet, daß** die Trägerpartikel Latexpartikel sind.

11. Testsystem nach einem der Ansprüche 1 bis 10, **dadurch gekennzeichnet, dass** die Trägerpartikel einzeln oder gruppenweise gleiche oder unterschiedliche Partikelgrößen aufweisen und/oder mit Fiuoreszenzstoffen unterschiedlicher Emissionsspektren und/oder unterschiedlicher Intensitäten markiert sind, so daß die Kodierung jeder Partikelpopulation aus der für sie spezifischen Kombination der Größe, Fluoreszenzemissionsspektrum und Fluoreszenzintensität besteht.

12. Testsystem nach einem der Ansprüche 1 bis 11, **dadurch gekennzeichnet, daß** mehrere Partikelpopulationen vorliegen, wobei alle Partikel die gleiche Größe aufweisen, alle Partikel mit demselben Fluoreszenzfarbstoff markiert sind, aber jede der Partikelpopulationen eine andere Fluoreszenzintensität aufweist.

13. Testsystem nach einem der Ansprüche 1 bis 12, **dadurch gekennzeichnet, daß** die Trägerpartikel eine Größe von 1 $\mu$m bis 10 $\mu$m aufweisen.

14. Testsystem nach einem der Ansprüche 1 bis 13, **dadurch gekennzeichnet, daß** es zusätzlich umfaßt:

    (i) für jeden der zu den Sondenantigenen komplementären Antikörper die Angabe einer Referenzkurve, welche das Verhältnis zwischen Antikörperkonzentration im Probenvolumen und gemessener Fluoreszenzsignalstärke wiedergibt und durch die Funktion:

$$Count = A + \frac{D - A}{1 + e^{B(C - \ln Konz.)}}$$

bzw.:

$$Konz. = \exp\{C - \ln[(D-A)/(Count - A) - 1]/B\}$$

mit:

Count = Median der gemessenen Fluoreszenzsignale

A = untere Asymptote der Referenzkurve
B = Steigung der Referenzkurve
C = Wendepunkt der Referenzkurve
D = obere Asymptote der Referenzkurve

beschriebenen ist, wobei die Parameter A, B, C, und D experimentell mittels Titration von Kalibratorseren ermitteltbar sind, und
(ii) ein Standardserum, welches alle zu den Sondenantigenen komplementären Antikörper in bekannten Konzentrationen enthält, und welches zur Rekalibrierung der Referenzkurve von jedem der Antikörper geeignet und vorgesehen ist.

**Claims**

1. Diagnostic test system for the detection of antibodies directed against pathogens of acute respiratory infections and atypical pneumonia, that comprises pathogens of acute respiratory infections and atypical pneumonia or antigenic fragments of these pathogens as antigen probes, wherein these antigen probes are coupled on carrier particles which are labelled individually or in groups, resulting in several particle populations, - each consisting of one or several particles of the same type, - and each being coded by a label specific for this group, namely the combination of size, fluorescence emission spectrum and fluorescence intensity **characterized in ,**

   - **that** the pathogens are selected from group I: Chlamydophila pneumoniae, Chlamydophila psittaci, Mycoplasma pneumoniae, Coxiella burnetii Phase 1 and 2, Legionella pneumophila, Bordetella pertussis, or from group II: Parainfluenza 1, 2, 3, RSV, Adenovirus, Influenza A and B, Picomaviren, Varizella-Zoster Virus, Measles Virus,
   - **that** the test system further comprises at least one marker for acute infection diseases and at least one marker for bacterial infections,
   - **that** the markers are also coupled on carrier particles,
   - and **that** each particle population is loaded with another type of antigen probe or marker.

2. Test system according to claim 1, **characterized in**
   **that** the pathogens selected from group I are Chlamydophila pneumoniae, Chlamydophila psittaci, Mycoplasma pneumoniae and Bordetella pertussis and in addition Chlamydia trachomatis.

3. Test system according to claim 2, **characterized in,**
   **that** it additionally comprises the pathogens Coxiella burnetii Phase 1 and 2 and Legionella pneumophila.

4. Test system according to claim 1, **characterized in**
   **that** the pathogens selected from group II are Parainfluenza 1, 2, 3, RSV, Adeno virus, Influenza A and B are applied.

5. Test system according to claim 4, **characterized in**
   **that** it additionally comprises the pathogens Varizella-Zoster virus and Measles Virus.

6. Test system according to claim 1, **characterized in**
   **that** the pathogens selected from group I are Chlamydophila pneumoniae and/or Chlamydophila psittaci Mycoplasma pneumoniae and Bordetella pertussis and that the pathogens selcted from group II are Parainfluenza 1, 2, 3, RSV and Adenovirus Influenza A and B and in addition Chlamydia trachomatis.

7. Test system according to one of claims 1 to 6, **characterized in that** Neopterin or CRP (=C - reactive protein) is applied as marker for acute infections.

8. Test system according to one of claims 1 to 7, **characterized in that** Procalcitonin or CRP (=C-reactive protein) is applied as marker for bacterial infections.

9. Test system according to one of claims 1 to 8, **characterized in that** it additionally comprises at least one marker for total IgA and/or at least one marker for total IgE.

10. Test system according to one of claims 1 to 9, **characterized in that** it the carrier particles are latex particles.

11. Test system according to one of claims 1 to 10, **characterized in that** the individual or groups of carrier particles have the same or different particle size and are labelled with fluorescent dyes of different emission spectrums and/or of different intensities, so that the coding of each particle population consists of a combination of size, fluorescent emission spectrum and fluorescence intensity which is specific for that particle population.

12. Test system according to one of claims 1 to 11, **characterized in that** several particle populations are available wherein all particles have the same size and are labelled with the same fluorescent dye but each particle population has a different intensity of fluorescence.

13. Test system according to one of claims 1 to 12, **characterized in that** the size of the carrier particles is 1 $\mu$m to 10 $\mu$m.

14. Test system according to one of claims 1 to 13, **characterized in that** it additionally comprises:

(i) for each antibody complementary to the antigen probes the indication of a reference curve, which reflects the relationship between antibody concentration in the probe volume and measured fluorescence signal strength and which is described by the function:

$$\text{Count} = A + \frac{D - A}{1 + e^{B(C - \ln \text{conc.})}}$$

or:

$$\text{Conc.} = \exp\{C - \ln[(D-A)/(\text{Count} - A) - 1]/B\}$$

with:

Count = Median of the measured fluorescence signal
A = lower asymptote of the reference curve
B = slope of the reference curve
C = turning point of the reference curve
D = upper asymptote of the reference curve

wherein the parameters A, B, C, and D may experimentally be determined by titration of calibration serums, and (ii) a standard serum that contains all antibodies which are complementary to the antigen probes in known concentrations and that is suitable and intended for recalibration of the reference curve of each antibody.

**Revendications**

1. Système de test diagnostique pour la détection d'anticorps contre les agents d'infections respiratoires aiguës et de pneumonies atypiques, lequel système présentent des agents d'infections respiratoires aiguës et de pneumonies atypiques ou des parties antigéniques de ces agents comme antigènes sonde, ces antigènes sonde étant couplés à des particules support qui isolées ou en groupe sont marquées de sorte que plusieurs populations de particules - composées chacune d'une ou plusieurs particules de même type - sont présentes, dont chacune est codée par un marquage spécifique pour elle, à savoir la combinaison de taille, spectre d'émission de fluorescence et intensité de fluorescence, **caractérisé**

- **en ce que** l'agent est choisi dans le groupe I : Chlamydophila pneumoniae, Chlamydophila psittaci, Mycoplasma pneumoniae, Coxiella burnetii phase 1 et 2, Legionella pneumophila, Bordetella pertussis, ou dans le groupe II : Parainfluenza 1, 2, 3, RSV, l'adénovirus, Influenza A et B, le picornavirus, le virus Varizella-Zoster, le virus de la rougeole,
- **en ce que** le système de test comprend en outre au moins un marqueur pour des maladies infectieuses aiguës et au moins un marqueur pour des infections bactériennes,
- **en ce que** les marqueurs sont eux aussi couplés à des particules porteuses,
- et **en ce que** chaque population de particule est chargée d'une sorte d'antigène sonde ou de marqueur.

2. Système de test selon la revendication 1, **caractérisé en ce qu'**on utilise comme agent du groupe I Chlamydophila pneumoniae, Chlamydophila psittaci, Mycoplasma pneumoniae et Bordetella pertussis et en plus Chlamydia trachomatis.

3. Système de test selon la revendication 2, **caractérisé en ce qu'**il comprend en outre les agents Coxiella burnetii phase 1 et 2 et Legionella pneumophila.

4. Système de test selon la revendication 1, **caractérisé en ce qu'**on utilise comme agents du groupe II Parainfluenza 1, 2, 3, RSV, l'adénovirus, Influenza A et B.

5. Système de test selon la revendication 4, **caractérisé en ce qu'**il comprend en outre les agents virus Varizella-Zoster et le virus de la rougeole.

6. Système de test selon la revendication 1, **caractérisé en ce que** sont utilisé comme agent du groupe I Chlamydophila pneumoniae et/ou Chlamydophila psittaci Mycoplasmas pneumoniae ainsi que Bordetella pertussis et comme agents du groupe II Parainfluenza 1, 2, 3, RSV ainsi que l'adénovirus Influenza A et B en en plus Chlamydia trachomatis.

7. Système de test selon l'une des revendications 1 à 6, **caractérisé en ce qu'**on utilise comme marqueur pou les infections aiguës de la néoptérine ou du CRP (= protéine C-réactive).

8. Système de test selon l'une des revendications 1 à 7, **caractérisé en ce qu'**on utilise comme marqueur pour les infections bactériennes de la procalcitonine ou du CRP (= protéine C-réactive).

9. Système de test selon l'une des revendications 1 à 8, **caractérisé en ce qu'**il comprend en outre au moins un marqueur pou l'IgA total et/ou au moins un marqueur pour IgE total.

10. Système de test selon l'une des revendications 1 à 10, **caractérisé en ce que** les particules support sont des particules de latex.

11. Système de test selon l'une des revendications 1 à 10, **caractérisé en ce que** les particules support isolées ou en groupe ont une taille de particule identique ou différente et/ou sont marquées avec des substances fluorescentes de spectres d'émission différents et/ou d'intensités différentes de sorte que le codage de chaque population de particule se compose de la combinaison propre à chacune de taille, spectre d'émission de fluorescence et intensité de fluorescence.

12. Système de test selon l'une des revendications 1 à 11, **caractérisé en ce que** plusieurs populations de particules sont présentes, toutes les particules ayant la même taille, toutes les particules sont marquées avec le même colorant de fluorescence, mais chacune des populations de particules présente une intensité de fluorescence différente.

13. Système de test selon l'une des revendications 1 à 12, **caractérisé en ce que** les particules support ont une taille comprise entre 1 $\mu$m et 10 $\mu$m.

14. Système de test selon l'une des revendications 1 à 13, **caractérisé en ce qu'**il comprend en outre :

(i) pour chacun des anticorps complémentaires des antigènes sonde l'indication d'une courbe de référence qui représente le rapport entre la concentration en anticorps dans le volume échantillon et l'intensité du signal de fluorescence mesurée et qui est décrite par la fonction :

$$\text{Count} = A + \frac{D - A}{1 + e^{B(C - \ln \text{Konz.})}}$$

respectivement

$$\text{Konz.} = \exp\{C - \ln[(D-A)/(\text{Count} -A)-1]/B\}$$

avec

Count = médiane des signaux de fluorescence mesurés
A = asymptote inférieure de la courbe de référence
B = gradient de la courbe de référence
C = point d'inflexion de la courbe de référence
D = asymptote supérieure de la courbe de référence

les paramètres A, B, C et D pouvant être déterminés de façon expérimentale par titration de sérums de calibration, et
(ii) un sérum standard qui comprend tous les anticorps complémentaires des antigènes sonde dans des concentrations connues et qui est apte et destiné au recalibrage de la courbe de référence de chacun des anticorps.

Fig. 1

Fig. 2

Fig. 3

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 3322373 C2
- EP 0126450 A
- DE 3639279 C3

### In der Beschreibung aufgeführte Nicht-Patentliteratur

- **MARTINS T.B.** *CLINICAL AND DIAGNOSTIC LABORATORY IMMUNOLOGY,* 2002, vol. 9 (1), 41-45
- **GENDREL et al.** *Pediatr Infect Dis J,* 1999, vol. 18 (10), 875-881
- **VAN DISSEL.** *Clin Microbiol Infect,* 2002, vol. 8, 70-73
- **CHAUHAN et al.** *Indian J Med res [B] 92,* 1990, 241-245
- **AUS BALDACCI et al.** *Eur Respir J,* 2001, vol. 17, 773-790
- **OLDACH et al.** *Clinical Infectious Dieseases,* 1993, vol. 17, 338-343
- **VAN LOON ; VAN DER VEEN.** *J. Clin. Pathol.,* 1980, vol. 33, 635-639
- **VAN LOON et al.** *J. Clin. Pathol.,* 1981, vol. 34, 665-669
- **DUNDLEY, R.A. ; EDWARDS, P. ; EKINS, R.P. ; FINNEY, D.J. ; MCKENZIE, J.G.M. ; RAAB, G.M. ; RODBARD, D. ; RODGERS, R.P.C.** Guideline for immunoassay data processing. *Clin. Chem.,* 1985, vol. 31, 1264-1271